(19) Europäisches Patentamt / European Patent Office / Office européen des brevets

(11) **EP 3 512 056 B1**

## (12) FASCICULE DE BREVET EUROPEEN

(45) Date de publication et mention de la délivrance du brevet:
**13.05.2020   Bulletin 2020/20**

(21) Numéro de dépôt: **18205804.0**

(22) Date de dépôt: **13.11.2018**

(51) Int Cl.:
*H02B 1/28* *(2006.01)*

(54) **PROCEDE DE DETECTION D'UN DYSFONCTIONNEMENT ELECTRIQUE, DISPOSITIF POUR LA MISE EN OEUVRE D'UN TEL PROCEDE ET ENCEINTE ELECTRIQUE EQUIPEE D'UN TEL DISPOSITIF**

VERFAHREN ZUM ERKENNEN EINER ELEKTRISCHEN STÖRUNG, VORRICHTUNG ZUR UMSETZUNG DIESES VERFAHRENS UND ELEKTRISCHES GEHÄUSE, DAS MIT EINER SOLCHEN VORRICHTUNG AUSGESTATTET IST

METHOD FOR DETECTING ELECTRICAL MALFUNCTION, DEVICE FOR IMPLEMENTING SUCH A METHOD AND ELECTRICAL ENCLOSURE EQUIPPED WITH SUCH A DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **16.01.2018   FR 1850336**

(43) Date de publication de la demande:
**17.07.2019   Bulletin 2019/29**

(73) Titulaire: **Schneider Electric Industries SAS**
**92500 Rueil Malmaison (FR)**

(72) Inventeurs:
  • **BONNARD, Frédéric**
**38050 Grenoble (FR)**
  • **MOUSTROU, Daniel**
**Cedex 09 Grenoble (FR)**
  • **BOUMEDIENE, Emir**
**38050 Grenoble (FR)**
  • **NEYRET, Yannick**
**38050 Grenoble (FR)**

(74) Mandataire: **Tripodi, Paul**
**Schneider Electric Industries SAS**
**Service Propriété Industrielle**
**World Trade Center / 38EE1**
**5 Place Robert Schuman**
**38050 Grenoble Cedex 09 (FR)**

(56) Documents cités:
**WO-A1-2010/043272      FR-A1- 2 509 922**
**US-A1- 2017 372 597**

## Description

### DOMAINE TECHNIQUE

La présente invention concerne un procédé pour détecter un dysfonctionnement dans une enceinte électrique telle qu'un tableau ou une armoire électrique. L'invention concerne également un dispositif pour détecter un dysfonctionnement dans une enceinte électrique et une enceinte électrique équipée d'un tel dispositif.

### ETAT DE LA TECHNIQUE

Aujourd'hui, de plus en plus de capteurs sont utilisés pour donner des informations sur la qualité de l'air que nous respirons ou encore pour détecter des fumées potentiellement dangereuses dans le cas d'un début d'incendie. Ce type d'information est utile dans les installations électriques ou l'exploitant a parfois la perception d'un échauffement anormal bien avant l'apparition d'une quelconque manifestation visible.

Le document US 6 317 053 B1 décrit une armoire électrique étanche à l'air ambiant, destinée à contenir du matériel informatique et comportant un dispositif de détection précoce d'incendie. Le dispositif comporte des détecteurs d'incendie placés à proximité d'un ventilateur d'extraction d'air chaud. Toute détection d'échauffement par un des capteurs déclenche l'injection d'un gaz inerte dans l'armoire électrique. Ce dispositif présente l'inconvénient de forcer l'air de l'armoire électrique à passer dans une canalisation pour pouvoir détecter un départ d'incendie. D'autre part, un tel dispositif n'a pas nécessité d'être discriminant, le fonctionnement naturel du matériel informatique ne génère pas de pollution pouvant être interprétée comme le résultat d'une combustion.

Le document EP 1 768 074 A1 décrit un dispositif de détection prompte d'incendie utilisant un capteur de fumée ou particules, un capteur de température et un capteur permettant de mesurer la vitesse du flux d'air autour du dispositif de détection d'incendie. La surveillance de la vitesse du flux d'air permet de détecter plus rapidement un incendie par rapport à un capteur d'incendie conventionnel. En revanche, le dispositif peut donner une fausse alarme si l'air est mis en mouvement par exemple par un ventilateur.

Le document WO 2010/043 272 décrit un détecteur multi-fonctions utilisé en gestion du bâtiment. Il comporte de multiples capteurs destinés à l'analyse de la qualité de l'air dans le bâtiment. Le document cite, entre autres, des capteurs de gaz (dioxyde de carbone, ozone, oxyde d'azote) comprenant des gaz inflammables (propane, butane, méthane, gaz naturel), des capteurs de particules, de fumée, de particules inflammables, d'amiante, acariens ou spores. Le détecteur émet une alarme en cas de détection d'une situation dangereuse pour la santé des occupants.

D'autres documents décrivent des solutions pour éviter la difficulté récurrente des fausses alarmes incendie. Le document EP 0 660 282 B1 décrit un système d'avertissement d'incendie utilisant un traitement des données issues des capteurs par la logique floue (« fuzzy logic »). Le document EP 0 141 987 B1 décrit un dispositif procédant à une validation des indications d'un détecteur après deux intervalles de temps et une remise en état initial de l'indicateur.

Il n'existe cependant pas de dispositif permettant de détecter un dysfonctionnement à l'origine d'un échauffement anormal de matériels électriques afin de donner une alerte précoce.

### EXPOSE DE L'INVENTION

La présente invention propose un procédé permettant de détecter l'émanation de composés caractéristiques liés à un échauffement anormal à l'intérieur d'une enceinte électrique. Un traitement particulier, basé sur plusieurs types de mesures, permet de détecter, de manière précoce et de façon fiable, une anomalie de fonctionnement se caractérisant par un échauffement anormal, même localisé à proximité de matériels dégageant de la chaleur.

Pour cela, l'invention concerne un procédé de détection d'un dysfonctionnement dans une enceinte électrique comportant au moins un équipement électrique et au moins un capteur de composés organiques volatiles, au moins un capteur de microparticules, au moins un capteur de gaz, ledit procédé comportant cycliquement dans le temps:

- la mesure d'au moins un paramètre climatique dans l'enceinte électrique,
- la mesure d'une concentration en composés organiques volatiles,
- la mesure d'une concentration en gaz,
- la mesure d'une concentration en microparticules,
- la correction des mesures de concentration en composés organiques volatiles, en gaz et en microparticules en fonction du, au moins un, paramètre climatique,
- le calcul d'une dérive de concentration en composés organiques volatiles, en gaz et en microparticules en fonction du temps,
- la comparaison de la dérive de concentration en composés organiques volatiles, en gaz et en microparticules respectivement à un seuil prédéfini de dérive de concentration en composés organiques volatiles, en gaz et en microparticules,
- le calcul d'une évolution de concentration en composés organiques volatiles, en gaz, et en microparticules,
- la comparaison de l'évolution de concentration en composés organiques volatiles, en gaz, et en microparticules à respectivement un seuil prédéfini d'évolution de concentration en composés organiques volatiles, en gaz, et en microparticules,
- l'émission d'une alarme quand :
- le seuil de dérive de concentration en microparticules ou le seuil d'évolution de concentration en microparticules est dépassé, et quand
- au moins un seuil de dérive de concentration ou un seuil d'évolution de concentration en composés organiques volatiles ou en gaz est dépassé.

Préférentiellement, le calcul de dérive de concentration en composés organiques volatiles, en gaz et en microparticules comporte :

- un calcul d'une moyenne glissante sur une période longue de la concentration en composés organiques volatiles, en gaz et en microparticules,
- un calcul d'une moyenne glissante sur une période courte, de la concentration en composés organiques volatiles, en gaz et en microparticules,
- un calcul d'un rapport respectivement entre la moyenne glissante sur la période courte et la moyenne glissante sur la période longue.

De préférence, la période courte est comprise entre 15 et 60 minutes et la période longue est comprise entre 5 et 12 heures.

Préférentiellement, le calcul de l'évolution de concentration respectivement en composés organiques volatiles, en gaz et en microparticules comporte au moins un calcul de la différence entre deux mesures consécutives de concentration respectivement en composés organiques volatiles, en gaz et en microparticules.

L'invention concerne également un dispositif de détection d'un dysfonctionnement électrique comportant :

- au moins un capteur pour fournir un signal caractéristique de la concentration en composés organiques volatiles,
- au moins un capteur pour fournir un signal caractéristique de la concentration en microparticules,
- au moins un capteur pour fournir un signal caractéristique de la concentration en gaz,
- au moins un capteur de paramètre climatique pour fournir une valeur d'au moins un paramètre climatique,
- un circuit de mesure pour mesurer les signaux fournis par les capteurs,
- un circuit d'alarme pour générer une alarme, et
- une unité de traitement comportant des circuits pour exécuter le procédé de détection d'un dysfonctionnement tel que décrit précédemment et pour activer le circuit d'alarme quand :
- le seuil de dérive de concentration en microparticules ou le seuil d'évolution de concentration en microparticules est dépassé, et quand
- au moins un seuil de dérive de concentration ou d'évolution de concentration en composés organiques volatiles ou en gaz est dépassé.

De préférence, le capteur de gaz fournit un signal caractérisant une concentration en ammoniaque.

Préférentiellement, le capteur de gaz fournit un signal caractérisant une concentration en ozone.

Préférentiellement, le capteur de composés organiques volatiles fournit un signal caractérisant une concentration en hydrocarbures.

De préférence, le capteur de paramètre climatique fournit un signal caractérisant une température dans l'enceinte électrique.

Préférentiellement, le capteur de paramètre climatique fournit, en outre, un signal caractérisant une hygrométrie dans l'enceinte électrique.

L'invention concerne également une enceinte électrique comportant au moins un câble ou un équipement électrique et un dispositif de détection d'un dysfonctionnement électrique tel que décrit précédemment.

**BREVE DESCRIPTION DES DESSINS**

D'autres avantages et caractéristiques ressortiront plus clairement de la description qui va suivre, de modes particuliers de réalisation de l'invention, donnés à titre d'exemples non limitatifs, et représentés aux dessins annexés sur lesquels :

- la figure 1 montre un organigramme d'un procédé de détection d'un dysfonctionnement à partir de mesures de conditions climatiques et de mesure de concentration en composés organiques volatiles, en microparticules et en gaz selon l'invention,
- la figure 2 représente un organigramme d'une partie du procédé représenté en figure 1 pour détailler un traitement de la mesure de concentration en gaz,
- la figure 3 représente un organigramme d'une partie du procédé représenté en figure 1 pour détailler un traitement de la mesure de concentration en microparticules,
- la figure 4 représente un organigramme d'une partie du procédé représenté en figure 1 pour détailler un traitement préférentiellement utilisé pour quantifier une évolution de concentration en microparticules,
- la figure 5 est un graphique représentant des mesures et des résultats de calculs pour illustrer un exemple de détection d'une dérive de concentration en composés organiques volatiles supérieure à un seuil de dérive prédéfini,
- la figure 6 est un graphique représentant des mesures et des résultats de calculs pour illustrer un exemple de détection d'une évolution supérieure à un seuil d'évolution de concentration en composés organiques volatiles prédéfini, et
- la figure 7 est un schéma bloc d'une enceinte électrique comportant au moins un câble ou un équipement électrique et un dispositif de détection d'un dysfonctionnement électrique selon l'invention.

## DESCRIPTION DETAILLEE DE MODES DE REALISATION PREFERES

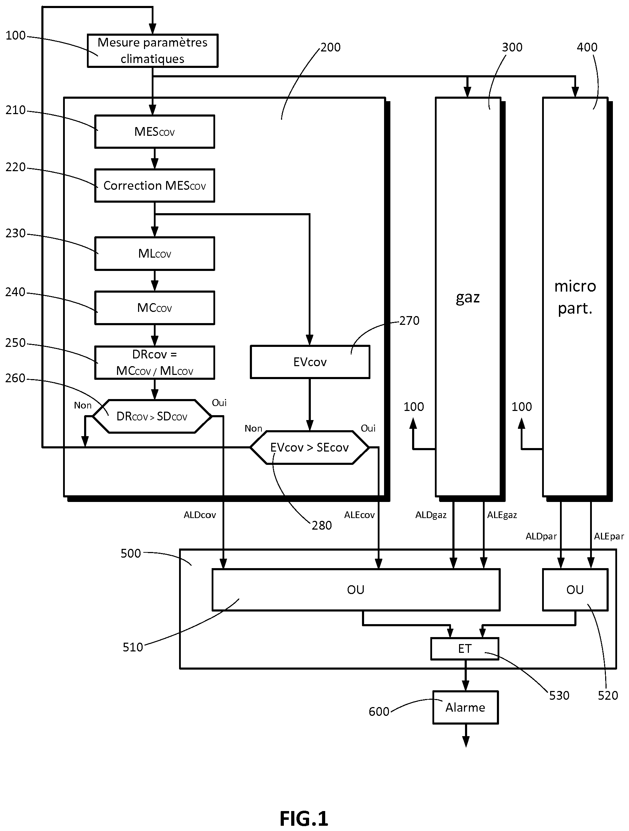

La figure 1 illustre un procédé de détection d'un dysfonctionnement dans une enceinte électrique 10 sous la forme d'un organigramme. Une mesure d'au moins un paramètre climatique dans l'enceinte électrique 10 est effectuée au cours d'une étape 100. Préférentiellement, deux paramètres climatiques sont mesurés: la température T et l'hygrométrie H. D'autres paramètres climatiques peuvent être mesurés, par exemple la pression atmosphérique P. La mesure d'au moins un de ces paramètres climatiques est nécessaire pour corriger les mesures effectuées par des capteurs de microparticules, de gaz et de composés organiques volatiles (COV) décrits ultérieurement. En effet, ces capteurs sont étalonnés en usine à une température et hygrométrie connue mais lesdits capteurs sont généralement sensibles aux conditions climatiques du milieu environnant. Le procédé se poursuit par des mesures de concentration en composés organiques volatiles (COV), microparticules et gaz dans l'atmosphère de l'enceinte électrique.

Au cours d'une étape 210, une mesure $MES_{COV}$ de concentration en composés organiques volatiles (COV) est effectuée au moyen d'un capteur de composés organiques volatiles 21, puis une étape de correction de la mesure 220 est effectuée pour corriger la mesure $MES_{COV}$ effectuée à l'étape 210 en fonction de la valeur du ou des paramètres climatiques mesurés à l'étape 100.

Dans une enceinte contenant des équipements électriques, il peut y avoir un ou plusieurs équipements 11, 12 diffusant des composés organiques volatiles en fonctionnement normal. Le procédé de l'invention est destiné à détecter précocement une émission de COV anormale, consécutive à un dysfonctionnement d'un équipement 11, 12, se superposant à une émission naturelle de COV d'un équipement 11, 12 en fonctionnement normal, c'est-à-dire sans anomalie. Pour cela, au cours d'une étape 230, le procédé effectue un calcul d'une moyenne $ML_{COV}$ de concentration en COV dans l'enceinte électrique 10 sur une période longue PL. Ladite moyenne de concentration $ML_{COV}$ reflète le niveau en fonctionnement normal de la concentration en COV à l'intérieur de l'enceinte 10. Pour être représentative du fonctionnement normal d'équipements électriques de la taille d'une armoire électrique, la période longue PL est préférentiellement comprise entre 5 et 12 heures. La période longue PL peut être ajustée en fonction de l'inertie thermique des équipements présents dans l'enceinte et/ou de la taille de l'enceinte 10.

De préférence, la moyenne de concentration sur la période longue $ML_{COV}$ est une moyenne glissante : les mesures de concentration en composés organiques volatiles $MES_{COV}$ sont effectuées à intervalle fixe, préférentiellement toutes les 30 secondes. Pour une période longue PL d'une durée préférentielle de 8 heures, la moyenne de concentration sur une période longue $ML_{COV}$ prendra en compte 960 mesures $MES_{COV}$. Toute nouvelle mesure $MES_{COV}$ remplace la plus ancienne, et un nouveau calcul de moyenne sur une période longue $ML_{COV}$ est effectué sur les 960 mesures $MES_{COV}$ les plus récentes.

Pour détecter précocement une émission de COV anormale d'un équipement 11, 12, un calcul de moyenne glissante de concentration en composés organiques volatiles $MC_{COV}$ sur une période courte PC, est effectué au cours d'une étape 240. Préférentiellement, la durée de la période courte est comprise entre 15 et 60 minutes. Ainsi, pour une période courte PC, de durée égale à 30 minutes, à raison d'une mesure $MES_{COV}$ de concentration effectuée de préférence toutes les 30 secondes, le calcul de moyenne sur la période courte $MC_{COV}$ effectuera une moyenne sur les 60 mesures $MES_{COV}$ les plus récentes. Ensuite, au cours d'une étape 250, un calcul de dérive $DR_{COV}$ de concentration en COV est effectué. La dérive de concentration en COV est égale au rapport de la moyenne de concentration en COV sur la période courte $MC_{COV}$ sur la moyenne de concentration en COV sur la période longue $ML_{COV}$. Ainsi, la dérive $DR_{COV}$ est égale à $MC_{COV} / ML_{COV}$. La dérive $DR_{cov}$ de concentration en COV est comparée à l'étape 260 à un seuil de dérive $SD_{COV}$ prédéfini. Quand la dérive $DR_{cov}$ est supérieure à $SD_{COV}$, il s'agit d'un dépassement anormal de la concentration en composés organiques volatiles et une alerte de dérive $ALD_{COV}$ de concentration en COV est émise. Ainsi, toute évolution anormale de concentration en COV pourra être détectée, dans le cas de figure ainsi décrit, dans les 30 minutes qui suivent l'apparition du dysfonctionnement. Préférentiellement, le seuil de dérive $SD_{COV}$ est compris entre 1,001 et 1,10 (entre 100,1% et 110% si le seuil est exprimé en pourcentage).

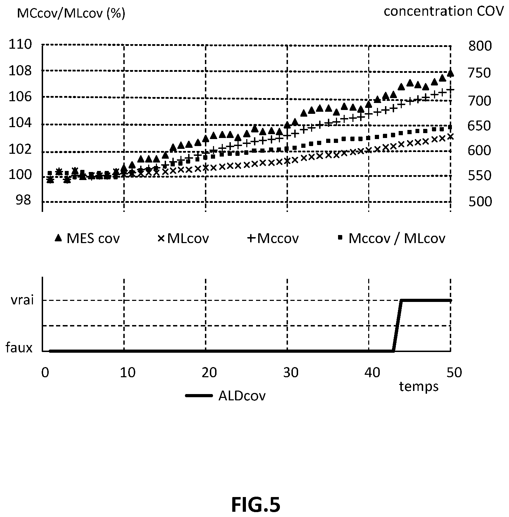

La figure 5 illustre, au moyen d'un graphique, un exemple de détection d'une dérive de concentration en COV. L'unité de temps correspond à une période de 30 secondes. Un dysfonctionnement apparait au temps t = 10. La courbe MEScov représente les valeurs de concentration mesurées en fonction du temps, la courbe $ML_{COV}$ représente le résultat du calcul de la moyenne longue et $MC_{COV}$ représente le résultat du calcul de la moyenne courte. Une graduation en concentration est indiquée pour ces trois courbes sur l'échelle droite du graphe. La courbe $MC_{COV}$ / $ML_{COV}$ représente le résultat du calcul de dérive DRcov, exprimé en pourcentage sur l'échelle gauche du graphe. La dérive DRcov dépasse un seuil de dérive $SD_{COV}$ prédéfini à la valeur de 1,03 (ou 103%) au temps t = 44 et une alerte ALDcov est générée. Le dysfonctionnement a donc été détecté dans un délai de 34 intervalles de mesure soit 17 minutes après le début du dysfonctionnement quand une mesure est effectuée toutes les 30 secondes. Les étapes de calcul de la dérive de concentration en COV dans l'enceinte électrique permettent de détecter un dysfonctionnement à l'origine d'un dégagement de COV se développant lentement, dans une atmosphère comportant déjà une concentration en COV pouvant être importante en régime normal.

Le procédé est destiné à détecter également un dysfonctionnement qui apparait et se développe rapidement. Pour cela, une étape 270 de calcul de l'évolution d'émission de composés organiques volatiles $EV_{COV}$ est effectuée. $EV_{COV}$ est calculé en effectuant la différence entre deux mesures $MES_{COV}$ consécutives. Soit $MES_{COV}$ (t) une mesure de $MES_{COV}$ à l'instant t et $MES_{COV}$ (t+1) une mesure de $MES_{COV}$ à l'instant t+1, alors, à l'instant t+1 :

$$EV_{COV} (t+1) = MES_{COV} (t+1) - MES_{COV} (t).$$

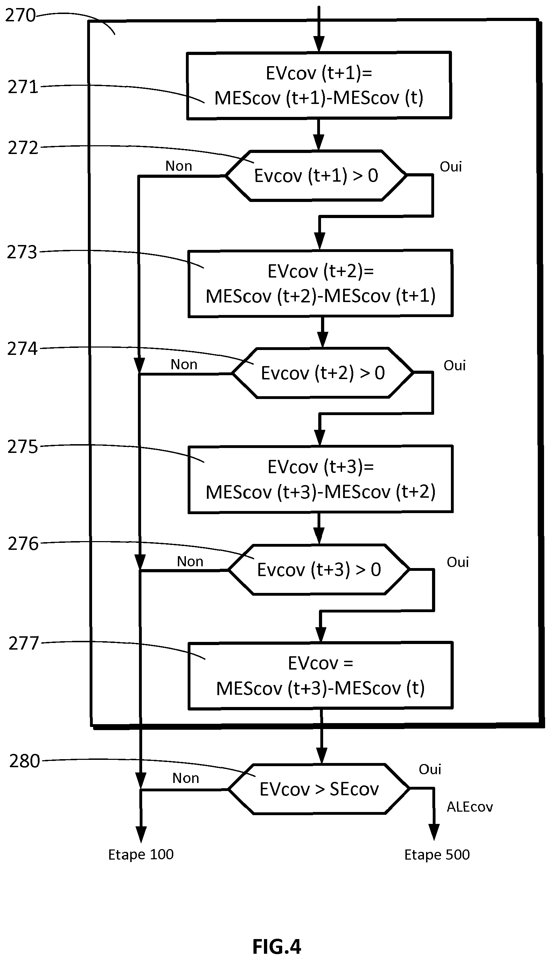

Le résultat de calcul de l'évolution d'émission $EV_{COV}$ (t+1) est comparé, à l'étape 280, à un seuil prédéfini d'évolution de concentration $SE_{COV}$. Quand le résultat de calcul de l'évolution d'émission $EV_{COV}$ (t+1) est supérieur au seuil d'évolution $SE_{COV}$ alors une alerte d'évolution $ALE_{COV}$ de concentration en COV est émise. Quand la valeur de $EV_{cov}$ (t+1) est négative ou inférieure au seuil prédéfini d'évolution $SE_{cov}$, le procédé retourne à la mesure 100 des paramètres climatiques. Afin d'éviter une fausse alarme, il est utile de vérifier que l'alerte est confirmée sur plusieurs cycles de mesures consécutifs. Selon un mode de réalisation préférentiel, quand tous les résultats de calcul de $EV_{COV}$ sont positifs sur 4 mesures consécutives de $EV_{COV}$ et que l'écart entre la dernière mesure effectuée $EV_{COV}$ (t+3) à l'instant t+3 et la première mesure $EV_{COV}$ (t) à l'instant t est supérieure au seuil $SE_{COV}$ alors l'alerte d'évolution $ALE_{COV}$ est émise. Un tel calcul itératif est illustré par un organigramme en figure 4. L'étape 270 de calcul de l'évolution d'émission de composés organiques volatiles $EV_{COV}$ commence par une étape 271 de calcul d'écart $EV_{COV}$ (t+1) entre la mesure $MES_{COV}$ (t) à l'instant t et la mesure $MES_{COV}$ (t+1) à l'instant (t+1). A l'étape 272, si l'évolution a été positive, c'est à dire si $EV_{COV}$ (t+1) >0, alors le procédé se poursuit par l'étape 273. Si EVCOV (t+1) est négatif, l'évolution est négative et il n'y a pas lieu de générer une alerte, le procédé retourne à l'étape 100 de mesure des paramètres climatiques. A l'étape 273, un calcul $EV_{COV}$ (t+2) = $MES_{COV}$ (t+2) - $MES_{COV}$ (t+1) est effectué. A l'étape 274, si $EV_{COV}$ (t+2) >0, alors le procédé se poursuit par l'étape 275 sinon le procédé retourne à l'étape 100. Les étapes 275 et 276 sont similaires aux étapes 273 et 274 mais s'appliquent aux mesures $MES_{COV}$ (t+3). Enfin à l'étape 276, quand $EV_{COV}$ (t+3) est positif, le procédé a détecté trois évolutions positives consécutives de $EV_{COV}$. Un calcul d'écart $EV_{COV}$ entre la mesure MESCOV (t+3) à l'instant (t+3) et la mesure $MES_{COV}$ (t) à l'instant t est effectué. Si $EV_{COV}$ est supérieur au seuil d'évolution $SE_{COV}$ alors une alerte d'évolution $ALE_{COV}$ de concentration en COV est émise. D'autres variantes de calcul de $EV_{COV}$ peuvent être utilisées.

Préférentiellement, le seuil d'évolution $SE_{COV}$ est compris entre 10 et 30 ppm.

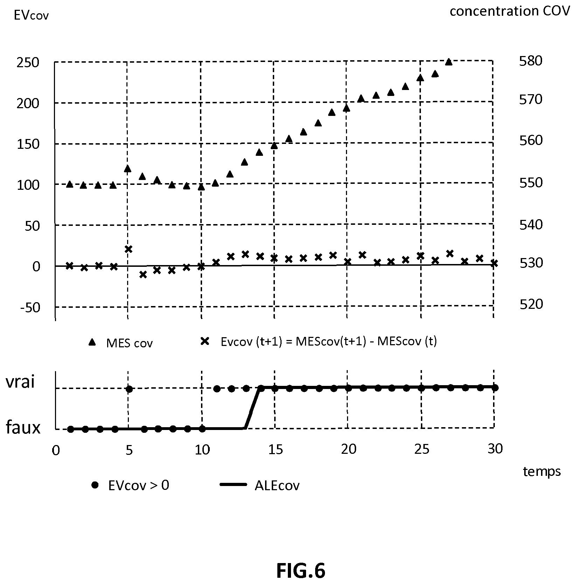

La figure 6 illustre au moyen d'un graphique, un exemple de détection d'une évolution anormale de concentration en COV selon le mode de réalisation préférentiel décrit précédemment. La courbe $MES_{COV}$ représente les valeurs de concentration mesurées en fonction du temps. Une graduation en concentration est indiquée sur l'échelle à droite du graphe. La courbe $EV_{COV}$ représente l'écart entre deux mesures $MES_{COV}$ consécutives. Une graduation à gauche du graphe correspond à la courbe EVcov. La courbe $EV_{COV}$ > 0 indique toutes les occurrences ou l'écart de valeur entre deux mesures consécutives est positive. Au temps t = 5, l'écart entre deux mesures consécutives est positif mais le phénomène ne s'est pas reproduit, l'alerte n'a donc pas été donnée. En revanche, à partir de t = 10, quatre dépassements consécutifs positifs ont eu lieu et le seuil SEcov a été dépassé, une alerte $ALE_{COV}$ est donc émise. Le dysfonctionnement qui s'est développé rapidement a donc été détecté dans un délai de 4 intervalles de mesure après le début du dysfonctionnement soit 2 minutes quand l'intervalle entre deux mesures est de 30 secondes.

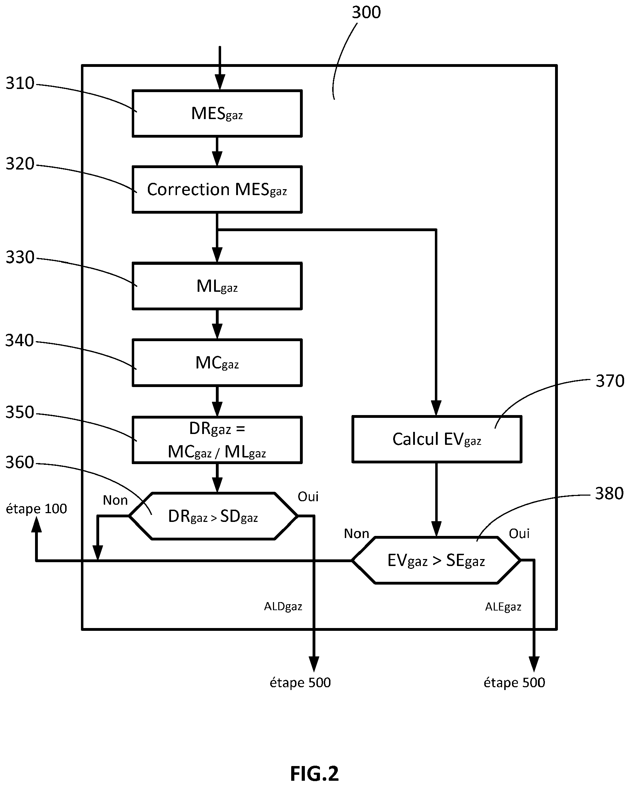

L'ensemble des étapes 210 à 280 de mesure de concentration en composés organiques volatiles (COV) et de détection de dépassement du seuil de dérive $SD_{COV}$ ou d'évolution de concentration $EV_{COV}$ fait partie d'un premier procédé 200 de mesure de concentration en composés organiques volatiles (COV). Un deuxième procédé 300 de mesure de concentration en gaz est également exécuté. Le deuxième procédé 300 est représenté dans l'organigramme de la figure 1 et de manière détaillée en figure 2. Le deuxième procédé 300 comporte des étapes similaires au premier procédé 200. Une étape 310 de mesure $MES_{gaz}$ de concentration en gaz est effectuée suivie par une étape 320 de correction de la mesure $MES_{gaz}$ en fonction du ou des paramètres climatiques mesurés à l'étape 100. Ensuite, une étape 330 est effectuée pour le calcul d'une moyenne glissante de concentration en gaz $ML_{gaz}$ dans l'enceinte électrique 10 sur la période longue PL puis une étape 340 est effectuée pour un calcul d'une moyenne glissante $MC_{gaz}$ sur la période courte PC. Un calcul d'une dérive de concentration en gaz DRgaz égale au rapport $MC_{gaz}$ / $ML_{gaz}$ est effectué au cours d'une étape 350 puis le calcul de dérive DRgaz est comparé à un seuil de dérive en concentration en gaz $SD_{gaz}$ à l'étape 360. Quand le seuil de dérive en concentration en gaz $SD_{gaz}$ est dépassé, une alerte de dérive de concentration en gaz $ALD_{gaz}$ est émise. Afin de détecter un dégagement de gaz se développant rapidement, un calcul de l'évolution de la concentration en gaz $EV_{gaz}$ est effectué selon des étapes similaires au calcul d'évolution de COV : au cours d'une étape 370, un calcul de l'évolution de concentration en gaz entre deux instants consécutifs t et t+1 est effectué selon l'équation $EV_{gaz}$ (t+1) = $MES_{gaz}$ (t+1) - $MES_{gaz}$ (t). A l'étape 380, la valeur de $EV_{gaz}$ (t+1) est comparée à un seuil prédéfini d'évolution $SE_{gaz}$. Quand la valeur $EV_{gaz}$ (t+1) est supérieure au seuil $SE_{gaz}$ alors une alerte d'évolution anormale de concentration en gaz $ALE_{gaz}$ est émise. Quand la valeur de $EV_{gaz}$ (t+1) est négative ou inférieure au seuil prédéfini d'évolution $SE_{gaz}$, le procédé retourne à la mesure 100 des paramètres climatiques. De préférence, le calcul de l'évolution de la concentration en gaz $EV_{gaz}$ est effectué selon un procédé similaire au calcul de l'évolution de concentration en COV portant sur quatre mesures consécutives positives. Préférentiellement, le seuil de dérive $SD_{gaz}$ est compris entre 1,001 et 1,1 (entre 100,1% et 110% exprimé en pourcentage) et le seuil d'évolution $SE_{gaz}$ est compris entre 5 et 50 ppm.

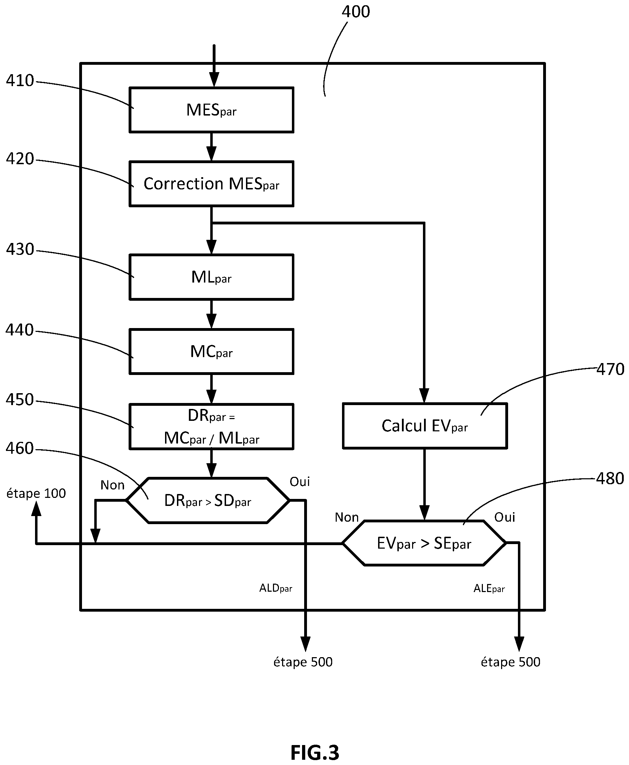

De la même façon, le procédé de détection de dysfonctionnement de l'invention comprend un troisième procédé 400 destiné à donner une alerte dans le cas d'une dérive ou d'une évolution anormale de la concentration en microparticules dans l'enceinte électrique. Le troisième procédé de mesure de concentration en microparticules 400 est représenté dans l'organigramme de la figure 1 et de manière détaillée en figure 3. Le troisième procédé 400 comporte des étapes similaires au premier procédé 200 et au deuxième procédé 300. Une étape 410 de mesure de la concentration en microparticules $MES_{par}$ est effectuée puis la mesure $MES_{par}$ est corrigée en fonction du ou des paramètres climatiques mesurés à l'étape 100, au cours d'une étape 420. Un calcul d'une moyenne glissante $ML_{par}$ de concentration en microparticules dans l'enceinte électrique 10 est effectué sur la période longue PL au cours d'une étape 430 et une moyenne glissante $MC_{par}$ est calculée sur la période courte PC au cours d'une étape 440. Un calcul d'une dérive de la concentration en microparticules $DR_{par}$ égale au rapport $MC_{par}$ / $ML_{par}$ est effectué dans une étape 450 et le résultat du calcul de dérive $DR_{par}$ est comparé à un seuil prédéfini de dérive en microparticules $SD_{par}$ au cours d'une étape 460. Quand le seuil de dérive en concentration en microparticules $SD_{par}$ est dépassé, une alerte de dérive de concentration en microparticule $ALD_{par}$ est émise. Si la valeur de la dérive $DR_{par}$ est inférieure au seuil prédéfini d'évolution $SD_{par}$, le procédé retourne à la mesure 100 des paramètres climatiques. Un calcul d'évolution de la concentration en microparticules $EV_{par}$ est également calculé : au cours d'une étape 470, un calcul de l'évolution de concentration en microparticules est effectué selon l'équation $EV_{par}$ (t+1) = $MES_{par}$ (t+1) - $MES_{par}$ (t). A l'étape 480, la valeur $EV_{par}$ est comparée à un seuil d'évolution prédéfini $SE_{par}$ afin de générer une alerte $ALE_{par}$ quand le résultat du calcul d'évolution de la concentration en microparticules $EV_{par}$ est supérieur au seuil d'évolution $SE_{par}$. Quand la valeur de $EV_{par}$ est négative ou inférieure au seuil d'évolution $SE_{par}$, le procédé retourne à la mesure 100 des paramètres climatiques. De préférence, le calcul de l'évolution de la concentration en microparticules $EV_{par}$ est effectué selon un procédé similaire au calcul de l'évolution de concentration en COV portant sur quatre mesures positives consécutives. Préférentiellement, le seuil de dérive $SD_{par}$ est compris entre 1 et 100 $\mu g/m^3$ et le seuil d'évolution $SE_{par}$ est compris entre 20 et 150 $\mu g/m^3$.

Les émissions de composés organiques volatiles, de microparticules ou de gaz sont liées au fonctionnement des équipements électriques présents dans l'enceinte électrique et ne sont pas obligatoirement un indicateur de dysfonctionnement. Une particularité de l'invention est la fourniture d'une alarme fiable concernant un dysfonctionnement d'un équipement en fonctionnement au milieu d'équipements sains. Pour cela, le procédé de détection de l'invention comporte une étape de concentration des informations d'alerte 500 au cours de laquelle est réalisés une opération logique « ET » entre les alertes en COV, en microparticules et en gaz. Une particularité de l'invention est de générer une alarme si au moins une alerte de concentration en microparticules est déclenchée et si au moins une alerte de concentration en COV ou en gaz est déclenchée. Ainsi qu'il est représenté en figure 1, d'une part, sous la référence 510, une opération logique « OU » des alertes de dérive et d'évolution pour les COV et les gaz est réalisée, d'autre part, sous la référence 520, une opération logique « OU » des alertes de dérive et d'évolution de concentration en microparticules est réalisé. Une opération logique « ET », sous la référence 530, est réalisée entre l'alerte de dépassement en microparticule et une alerte de dépassement en gaz ou en COV. Une équation logique de génération d'une variable alarme AL s'écrit :

$AL = (ALD_{par}$ OU $ALE_{par})$ ET $(ALD_{COV}$ OU $ALE_{COV}$ OU $ALD_{gaz}$ OU $ALE_{gaz})$

Quand la variable alarme AL est vraie, une alarme est générée au cours de l'étape 600. L'exploitant de l'installation électrique peut intervenir et remédier rapidement au dysfonctionnement.

A titre d'illustration de l'intérêt du procédé, une connexion mal serrée d'un câble de faible puissance sur une borne peut conduire à un échauffement hors spécification du matériau d'isolation du câble. Le matériau d'isolation surchauffé va émettre des COV, des gaz et des microparticules. D'autre part, des câbles de forte puissance, dans lesquels circulent des courant importants, peuvent être également présents dans l'enceinte. Les matériaux d'isolation des câbles de forte puissance émettent en fonctionnement normal des COV, gaz et microparticules, en quantités pouvant être largement supérieures à celles émises par le câble de faible puissance en surchauffe. Les étapes de calcul de dérive de la concentration en COV, microparticules et gaz vont permettre de déceler une variation positive et anormale de concentration en COV, gaz et microparticules, par rapport à un niveau correspondant à un fonctionnement normal. Un éventuel emballement thermique au niveau du câble de faible puissance sera détecté rapidement au cours des étapes de calcul d'évolution de la concentration. Un dépassement combiné de seuils de concentration en COV, microparticules et gaz permet de ne générer une alarme que lorsqu'il y a réel dysfonctionnement. Un tel procédé permet également de détecter un dysfonctionnement tel qu'un arc électrique anormal dans l'enceinte électrique 10 grâce à la détection des COV, microparticules et des gaz, en particulier l'ozone, émis par les matériaux soumis aux effets de l'arc électrique.

Le procédé de l'invention est exécuté cycliquement afin d'assurer une surveillance permanente des équipements dans l'enceinte électrique 10 et détecter toute évolution anormale en COV, gaz ou microparticules le plus rapidement possible. L'intervalle entre deux cycles consécutifs peut être compris entre quelques secondes et plusieurs minutes. Préférentiellement, ledit intervalle est égal à 30 secondes.

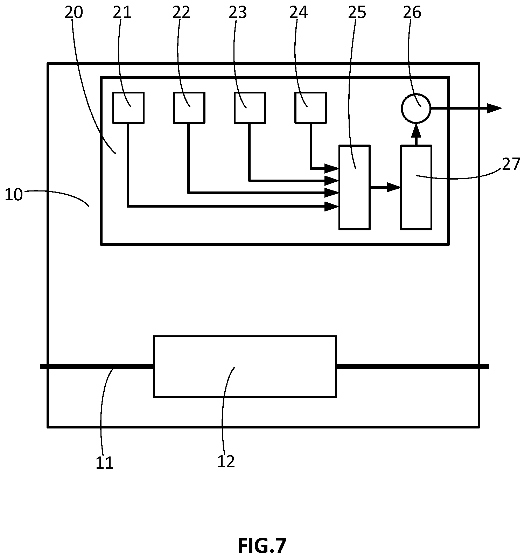

L'invention concerne également un dispositif 20, représenté en figure 7, pour détecter un dysfonctionnement dans une enceinte électrique 10. Le dispositif de détection 20 comporte :

- au moins un capteur 21 pour fournir un signal caractéristique de la concentration en composés organiques volatiles,
- au moins un capteur 22 pour fournir un signal caractéristique de la concentration en microparticules,
- au moins un capteur 23 pour fournir un signal caractéristique de la concentration en gaz,
- au moins un capteur de paramètre climatique 24,
- un circuit de mesure 25 pour mesurer les signaux fournis par les capteurs,
- un circuit d'alarme 26 pour générer une alarme, et
- une unité de traitement 27 comportant des circuits pour exécuter le procédé de détection d'un dysfonctionnement décrit précédemment, et pour activer le circuit d'alarme 26 quand le seuil de dérive de concentration en microparticules $SD_{par}$ ou le seuil d'évolution de concentration en microparticules $EV_{par}$ est dépassé, et quand au moins un seuil de dérive de concentration ou un seuil d'évolution de concentration en composés organiques volatiles SDcov, $EV_{COV}$ ou en gaz $SD_{gaz}$, $EV_{gaz}$ est dépassé. L'unité de traitement peut exécuter les premier, deuxième et troisième procédés de détection de dépassement du seuil de dérive ou d'évolution de concentration 200, 300 et 400, simultanément au moyen d'unités de calculs tels que des microprocesseurs fonctionnant en parallèle ou séquentiellement, l'un après l'autre s'il n'existe qu'une unité de calcul dans l'unité de traitement 27. Tout moyen de calcul tel qu'un automate, un assemblage de circuit électroniques logiques et/ou analogiques peut être utilisé. Le procédé de l'invention est exécuté cycliquement par l'unité de traitement, préférentiellement avec une période de 30 secondes. Selon un mode de réalisation préférentiel, les capteurs sont calibrés lors de la fabrication du dispositif de détection 20 et des courbes de correction en température et hygrométrie des signaux fournis par les capteurs de COV 21, de microparticules 22 et de gaz 23 sont mémorisées dans l'unité de traitement 27 pour l'exécution des étapes de correction 220, 320, 420 des mesures de concentration en composés organiques volatiles MEScov, en gaz MESgaz et en microparticules MESpar. Le capteur de paramètres climatiques 24 est préférentiellement un capteur de température et d'hygrométrie. D'autres capteurs 24 peuvent être ajoutés pour la mesure d'autres paramètres climatiques, par exemple la pression atmosphérique à l'intérieur ou à proximité de l'enceinte électrique 10.

Les composés organiques volatiles détectés préférentiellement par le capteur 21 sont composés ou comportent des molécules d'hydrocarbure ou d'alcool, de benzène, d'éthanol, de propane, d'iso-butane, de T-Butanol ou encore de 2-Butanone. Un capteur de COV 21 peut être réalisé par l'association de plusieurs capteurs spécifiques à certains types de COV et fournir un signal caractéristique de la concentration totale en composés organiques volatiles. Plusieurs capteurs peuvent également être utilisés et, dans ce cas, le circuit de mesure 25 effectue une somme, éventuellement pondérée, des signaux émis par chaque capteur pour élaborer la mesure MEScov.

Le capteur de microparticules 22 détecte préférentiellement les microparticules de taille comprise entre 1 et 5 microns émises par le ou les matériaux isolants des câbles électriques. Un capteur de microparticules 22 peut être réalisé par l'association de plusieurs capteurs spécifiques à certains types de microparticules et fournir un signal caractéristique de la concentration totale en microparticules. Plusieurs capteurs peuvent également être utilisés et, dans ce cas, le circuit de mesure 25 effectue une somme, éventuellement pondérée, des signaux émis par chaque capteur pour élaborer la mesure $MES_{par}$.

Les gaz détectés préférentiellement par le capteur de gaz 23 sont :

- l'ammoniaque,
- l'hydrogène,
- l'acétone
- l'ozone.

Un capteur de gaz 23 peut être réalisé par l'association de plusieurs capteurs spécifiques à certains gaz et fournir un signal caractéristique de la concentration totale en gaz. Plusieurs capteurs peuvent également être utilisés et, dans ce cas, le circuit de mesure 25 effectue une somme, éventuellement pondérée, des signaux émis par chaque capteur pour élaborer la mesure $MES_{gaz}$.

L'invention concerne aussi une enceinte électrique 10 comportant au moins un câble ou un équipement électrique 11, 12 et un dispositif de détection d'un dysfonctionnement électrique 20 pour détecter un échauffement anormal d'un câble ou d'un équipement électrique 11, 12. L'alarme générée par le circuit d'alarme 26 est préférentiellement émise au moyen d'un dispositif avertisseur lumineux et/ou sonore à proximité de l'enceinte électrique 10 et peut être simultanément transmise par des moyens filaires ou radio à une centrale de supervision distante non représentée sur la figure 7. Ainsi informé, un personnel peut intervenir rapidement pour supprimer la cause du dysfonctionnement.

## Revendications

1. Procédé de détection d'un dysfonctionnement dans une enceinte électrique (10) comportant au moins un équipement électrique (11, 12) et au moins un capteur de composés organiques volatiles (21), au moins un capteur de microparticules (22), au moins un capteur de gaz (23), ledit procédé comportant cycliquement dans le temps:

   - la mesure (100) d'au moins un paramètre climatique (T) dans l'enceinte électrique (10),
   - la mesure (210) d'une concentration en composés organiques volatiles ($MES_{COV}$),
   - la mesure (310) d'une concentration en gaz ($MES_{gaz}$),
   - la mesure (410) d'une concentration en microparticules ($MES_{par}$),
   - la correction (220, 320, 420) des mesures de concentration en composés organiques volatiles ($MES_{COV}$), en gaz ($MES_{gaz}$) et en microparticules ($MESpar$) en fonction du, au moins un, paramètre climatique (T),
   - le calcul d'une dérive de concentration (250, 350, 450) en composés organiques volatiles ($DR_{COV}$), en gaz ($DRgaz$) et en microparticules ($DRpar$) en fonction du temps,
   - la comparaison de la dérive de concentration (260, 360, 460) en composés organiques volatiles ($DR_{COV}$), en gaz ($DRgaz$) et en microparticules ($DRpar$) respectivement à un seuil prédéfini de dérive de concentration en composés organiques volatiles ($SD_{COV}$), en gaz ($SD_{gaz}$) et en microparticules ($SD_{par}$),
   - le calcul d'une évolution de concentration (270, 370, 470) en composés organiques volatiles ($EVcov$), en gaz ($EV_{gaz}$), et en microparticules ($EV_{par}$),
   - la comparaison de l'évolution de concentration (280, 380, 480) en composés organiques volatiles ($EV_{COV}$), en gaz ($EV_{gaz}$), et en microparticules ($EV_{par}$) à respectivement un seuil prédéfini d'évolution de concentration en composés organiques volatiles ($SE_{COV}$), en gaz ($SE_{gaz}$), et en microparticules ($SE_{par}$),
   - l'émission d'une alarme (600) quand :

      - le seuil de dérive de concentration en microparticules ($SD_{par}$) ou le seuil d'évolution de concentration en microparticules ($EV_{par}$) est dépassé, et quand
      - au moins un seuil de dérive de concentration ou un seuil d'évolution de concentration en composés organiques volatiles ($SD_{COV}$, $EVcov$) ou en gaz ($SDgaz$, $EVgaz$) est dépassé.

2. Procédé de détection d'un dysfonctionnement dans une enceinte électrique (10) selon la revendication 1 **caractérisé en ce que** le calcul (250, 350, 450) de dérive de concentration en composés organiques volatiles ($DR_{COV}$), en gaz ($DRgaz$) et en microparticules ($DRpar$) comporte :

   - un calcul d'une moyenne glissante sur une période longue (PL) de la concentration en composés organiques volatiles ($ML_{COV}$), en gaz ($ML_{GAZ}$) et en microparticules ($ML_{PAR}$),
   - un calcul d'une moyenne glissante sur une période courte (PC), de la concentration en composés organiques volatiles ($MC_{COV}$), en gaz ($MC_{GAZ}$) et en microparticules ($MC_{PAR}$),
   - un calcul d'un rapport respectivement entre la moyenne glissante sur la période courte ($MC_{COV}$, $MC_{GAZ}$, $MC_{PAR}$) et la moyenne glissante sur la période longue ($ML_{COV}$, $ML_{GAZ}$, $ML_{PAR}$).

3.  Procédé de détection d'un dysfonctionnement dans une enceinte électrique selon la revendication précédente **caractérisé en ce que** la période courte (PC) est comprise entre 15 et 60 minutes et la période longue (PL) est comprise entre 5 et 12 heures.

4.  Procédé de détection d'un dysfonctionnement dans une enceinte électrique (10) selon l'une des revendications précédentes **caractérisé en ce que** le calcul de l'évolution de concentration (270, 370, 470) respectivement en composés organiques volatiles ($EV_{COV}$), en gaz ($EV_{gaz}$) et en microparticules ($EV_{par}$) comporte au moins un calcul de la différence entre deux mesures consécutives de concentration respectivement en composés organiques volatiles (MEScov), en gaz (MESgaz) et en microparticules (MESpar).

5.  Dispositif de détection d'un dysfonctionnement électrique (20) comportant :

    - au moins un capteur (21) pour fournir un signal caractéristique de la concentration en composés organiques volatiles,
    - au moins un capteur (22) pour fournir un signal caractéristique de la concentration en microparticules,
    - au moins un capteur (23) pour fournir un signal caractéristique de la concentration en gaz,
    - au moins un capteur de paramètre climatique (24) pour fournir une valeur d'au moins un paramètre climatique (T),
    - un circuit de mesure (25) pour mesurer les signaux fournis par les capteurs (21, 22, 23, 24),
    - un circuit d'alarme (26) pour générer une alarme (AL), et
    - une unité de traitement (27),
    **caractérisé en ce que** l'unité de traitement (27) comporte des circuits adaptés pour exécuter le procédé de détection d'un dysfonctionnement selon les revendications 1 à 4 et pour activer le circuit d'alarme (26) quand :

        - le seuil de dérive de concentration en microparticules ($SD_{par}$) ou le seuil d'évolution de concentration en microparticules ($EV_{par}$) est dépassé, et quand
        - au moins un seuil de dérive de concentration ou d'évolution de concentration en composés organiques volatiles (SDcov, EVcov) ou en gaz (SDgaz, EVgaz) est dépassé.

6.  Dispositif de détection d'un dysfonctionnement électrique (20) selon la revendication précédente **caractérisé en ce que** le capteur de gaz (23) fournit un signal caractérisant une concentration en ammoniaque.

7.  Dispositif de détection d'un dysfonctionnement électrique (20) selon l'une des revendications 5 ou 6 **caractérisé en ce que** le capteur de gaz (23) fournit un signal caractérisant une concentration en ozone.

8.  Dispositif de détection d'un dysfonctionnement électrique (20) selon l'une quelconque des revendications 5 à 7 **caractérisé en ce que** le capteur de composés organiques volatiles (21) fournit un signal caractérisant une concentration en hydrocarbures.

9.  Dispositif de détection d'un dysfonctionnement électrique (20) selon l'une quelconque des revendications 5 à 8 **caractérisé en ce que** le capteur de paramètre climatique (24) fournit un signal caractérisant une température (T) dans l'enceinte électrique (10).

10. Dispositif de détection d'un dysfonctionnement électrique (20) selon la revendication 9 **caractérisé en ce que** le capteur de paramètre climatique (24) fournit, en outre, un signal caractérisant une hygrométrie (H) dans l'enceinte électrique (10).

11. Enceinte électrique (10) comportant au moins un câble ou un équipement électrique (11,12) **caractérisée en ce qu'**elle comporte un dispositif de détection (10) d'un dysfonctionnement électrique selon l'une quelconque des revendications 5 à 10.

**Patentansprüche**

1.  Verfahren zum Erkennen einer Störung in einem Schaltschrank (10), umfassend mindestens eine elektrische Ausrüstung (11, 12) und mindestens einen Sensor für flüchtige organische Verbindungen (21), mindestens einen Sensor für Mikropartikel (22), mindestens einen Gassensor (23), wobei das Verfahren zeitlich zyklisch umfasst:

- das Messen (100) mindestens einen Klimaparameters (T) in dem Schaltschrank (10),
- das Messen (210) einer Konzentration an flüchtigen organischen Verbindungen ($MES_{cov}$),
- das Messen (310) einer Gaskonzentration ($MES_{gaz}$),
- das Messen (410) einer Konzentration an Mikropartikeln ($MES_{par}$),
- die Korrektur (220, 320, 420) der Messungen einer Konzentration an flüchtigen organischen Verbindungen ($MES_{cov}$), an Gas ($MES_{gaz}$) und an Mikropartikeln ($MES_{par}$) in Abhängigkeit von dem mindestens einen Klimaparameter (T),
- das Berechnen einer Konzentrationsabweichung (250, 350, 450) an flüchtigen organischen Verbindungen ($DR_{cov}$), an Gas ($DR_{gaz}$) und an Mikropartikeln ($DR_{par}$) in Abhängigkeit von der Zeit,
- das Vergleichen der Konzentrationsabweichung (260, 360, 460) an flüchtigen organischen Verbindungen ($DR_{cov}$), an Gas ($DR_{gaz}$) und an Mikropartikeln ($DR_{par}$) jeweils mit einer vordefinierten Grenze einer Konzentrationsabweichung an flüchtigen organischen Verbindungen ($SD_{cov}$), an Gas ($SD_{gaz}$) und an Mikropartikeln ($SD_{par}$),
- das Berechnen einer einer Konzentrationsentwicklung (270, 370, 470) an flüchtigen organischen Verbindungen ($EV_{cov}$), an Gas ($EV_{gaz}$) und an Mikropartikeln ($EV_{par}$),
- das Vergleichen der Konzentrationsentwicklung (280, 380, 480) an flüchtigen organischen Verbindungen ($EV_{cov}$), an Gas ($EV_{gaz}$) und an Mikropartikeln ($EV_{par}$) mit einer vordefinierten Grenze einer Konzentrationsentwicklung an flüchtigen organischen Verbindungen ($SE_{cov}$), an Gas ($SE_{gaz}$) bzw. an Mikropartikeln ($SE_{par}$),
- das Senden eines Alarms (600), wenn:
- die Grenze einer Konzentrationsabweichung an Mikropartikeln ($SD_{par}$) oder die Grenze einer Konzentrationsentwicklung an Mikropartikeln ($EV_{par}$) überschritten wird, und wenn
- mindestens eine Grenze einer Konzentrationsabweichung oder eine Grenze einer Konzentrationsentwicklung an flüchtigen organischen Verbindungen ($SD_{cov}$, $EV_{cov}$) oder an Gas ($SD_{gaz}$, $EV_{gaz}$) überschritten wird.

2. Verfahren zum Erkennen einer Störung in einem Schaltschrank (10) nach Anspruch 1, **dadurch gekennzeichnet, dass** das Berechnen (250, 350, 450) einer Konzentrationsabweichung an flüchtigen organischen Verbindungen ($DR_{cov}$), an Gas ($DR_{gaz}$) und an Mikropartikeln ($DR_{par}$) umfasst:

- ein Berechnen eines gleitenden Mittelwerts über einen langen Zeitraum (PL) der Konzentration an flüchtigen organischen Verbindungen ($ML_{cov}$), an Gas ($ML_{gaz}$) und an Mikropartikeln ($ML_{par}$),
- ein Berechnen eines gleitenden Mittelwerts über einen kurzen Zeitraum (PC) der Konzentration an flüchtigen organischen Verbindungen ($MC_{cov}$), an Gas ($MC_{gaz}$) und an Mikropartikeln ($MC_{par}$),
- ein Berechnen eines Verhältnisses zwischen dem gleitenden Mittelwert über den kurzen Zeitraum ($MC_{cov}$, $MC_{gaz}$, $MC_{par}$) bzw. dem gleitenden Mittelwert über den langen Zeitraum ($ML_{cov}$, $ML_{gaz}$, $ML_{par}$).

3. Verfahren zum Erkennen einer Störung in einem Schaltschrank nach dem vorhergehenden Anspruch, **dadurch gekennzeichnet, dass** der kurze Zeitraum (PC) zwischen 15 und 60 Minuten beträgt, und der lange Zeitraum (PL) zwischen 5 und 12 Stunden beträgt.

4. Verfahren zum Erkennen einer Störung in einem Schaltschrank (10) nach einem der vorhergehenden Ansprüche, **dadurch gekennzeichnet, dass** das Berechnen einer Konzentrationsentwicklung (270, 370, 470) an flüchtigen organischen Verbindungen ($EV_{cov}$), an Gas ($EV_{gaz}$) bzw. an Mikropartikeln ($EV_{par}$) mindestens ein Berechnen der Differenz zwischen zwei aufeinanderfolgenden Konzentrationsmessungen an flüchtigen organischen Verbindungen ($MES_{cov}$), an Gas ($MES_{gaz}$) bzw. an Mikropartikeln ($MES_{par}$) umfasst.

5. Vorrichtung zum Erkennen einer elektrischen Störung (20), umfassend:

- mindestens einen Sensor (21), um ein für die Konzentration an flüchtigen organischen Verbindungen charakteristisches Signal zu liefern,
- mindestens einen Sensor (22), um ein für die Konzentration an Mikropartikeln charakteristisches Signal zu liefern,
- mindestens einen Sensor (23), um ein für die Gaskonzentration charakteristisches Signal zu liefern,
- mindestens einen Sensor für einen Klimaparameter (24), um einen Wert mindestens eines Klimaparameters (T) zu liefern,
- eine Messschaltung (25), um die von den Sensoren (21, 22, 23, 24) gelieferten Signale zu messen,
- eine Alarmschaltung (26), um einen Alarm (AL) zu erzeugen, und
- eine Bearbeitungseinheit (27),

**dadurch gekennzeichnet, dass** die Bearbeitungseinheit (27) Schaltungen umfasst, die dazu vorgesehen sind, das Verfahren zum Erkennen einer Störung nach den Ansprüchen 1 bis 4 auszuführen, und um die Alarmschaltung (26) zu aktivieren, wenn:

- die Grenze einer Konzentrationsabweichung an Mikropartikeln ($SD_{par}$) oder die Grenze einer Konzentrationsentwicklung an Mikropartikeln ($EV_{par}$) überschritten wird, und wenn
- mindestens eine Grenze einer Konzentrationsabweichung oder eine Grenze einer Konzentrationsentwicklung an flüchtigen organischen Verbindungen ($SD_{cov}$, $EV_{cov}$) oder an Gas ($SD_{gaz}$, $EV_{gaz}$) überschritten wird.

6. Vorrichtung zum Erkennen einer elektrischen Störung (20) nach dem vorhergehenden Anspruch, **dadurch gekennzeichnet, dass** der Gassensor (23) ein Signal liefert, das eine Ammoniak-Konzentration kennzeichnet.

7. Vorrichtung zum Erkennen einer elektrischen Störung (20) nach einem der Ansprüche 5 oder 6, **dadurch gekennzeichnet, dass** der Gassensor (23) ein Signal liefert, das eine Ozon-Konzentration kennzeichnet.

8. Vorrichtung zum Erkennen einer elektrischen Störung (20) nach einem der Ansprüche 5 bis 7, **dadurch gekennzeichnet, dass** der Sensor für flüchtige organische Verbindungen (21) ein Signal liefert, das eine Kohlenwasserstoff-Konzentration kennzeichnet.

9. Vorrichtung zum Erkennen einer elektrischen Störung (20) nach einem der Ansprüche 5 bis 8, **dadurch gekennzeichnet, dass** der Sensor für einen Klimaparameter (24) ein Signal liefert, das eine Temperatur (T) in dem Schaltschrank (10) kennzeichnet.

10. Vorrichtung zum Erkennen einer elektrischen Störung (20) nach Anspruch 9, **dadurch gekennzeichnet, dass** der Sensor für einen Klimaparameter (24) ferner ein Signal liefert, das eine Hygrometrie (H) in dem Schaltschrank (10) kennzeichnet.

11. Schaltschrank (10), umfassend mindestens ein Kabel oder eine elektrische Ausrüstung (11, 12), **dadurch gekennzeichnet, dass** er eine Vorrichtung (10) zum Erkennen einer elektrischen Störung nach einem der Ansprüche 5 bis 10 umfasst.

**Claims**

1. Method for detecting a fault in an electrical enclosure (10) comprising at least one electrical equipment item (11, 12) and at least one volatile organic compounds sensor (21), at least one microparticles sensor (22), at least one gas sensor (23), said method cyclically comprising over time:

- measuring (100) at least one climate parameter (T) in the electrical enclosure (10);
- measuring (210) a concentration of volatile organic compounds ($MES_{COV}$);
- measuring (310) a concentration of gases ($MES_{gas}$);
- measuring (410) a concentration of microparticles ($MES_{par}$);
- correcting (220, 320, 420) the measurements of the concentration of volatile organic compounds ($MES_{COV}$), of gases ($MES_{gas}$) and of microparticles ($MES_{par}$) on the basis of the at least one climate parameter (T);
- calculating (250, 350, 450) a drift of concentration of volatile organic compounds ($DR_{COV}$), of gases ($DR_{gas}$) and of microparticles ($DR_{par}$) on the basis of time;
- comparing (260, 360, 460) the drift of concentration of volatile organic compounds ($DR_{COV}$), of gases ($DR_{gas}$) and of microparticles ($DR_{par}$), respectively, to a predefined drift of concentration threshold of volatile organic compounds ($SD_{COV}$), of gases ($SD_{gas}$) and of microparticles ($SD_{par}$);
- calculating (270, 370, 470) a change of concentration of volatile organic compounds ($EV_{COV}$), of gases ($EV_{gas}$) and of microparticles ($EV_{par}$);
- comparing (280, 380, 480) the change of concentration of volatile organic compounds ($EV_{COV}$), of gases ($EV_{gas}$) and of microparticles ($EV_{par}$), respectively, to a predefined change of concentration threshold of volatile organic compounds ($SEvoc$), of gases ($SE_{gas}$) and of microparticles ($SE_{par}$);
- emitting an alarm (600) when:
- the drift of concentration threshold of microparticles ($SD_{par}$) or the change of concentration threshold of microparticles ($EV_{par}$) is exceeded; and when
- at least one drift of concentration threshold or one change of concentration threshold of volatile organic compounds ($SD_{VOC}$, $EV_{VOC}$) or of gases ($SD_{gas}$, $EV_{gas}$) is exceeded.

2. Method for detecting a fault in an electrical enclosure (10) according to Claim 1, **characterized in that** calculating (250, 350, 450) a drift of concentration of volatile organic compounds ($DR_{COV}$), of gases ($DR_{gas}$) and of microparticles ($DR_{par}$) comprises:

- calculating a running mean, over a long period (LP), of the concentration of volatile organic compounds ($ML_{VOC}$), of gases ($ML_{GAS}$) and of microparticles ($ML_{PAR}$);
- calculating a running mean, over a short period (SP), of the concentration of volatile organic compounds ($MC_{VOC}$), of gases ($MC_{GAS}$) and of microparticles ($MC_{PAR}$);
- calculating a ratio respectively between the running mean over the short period ($MC_{VOC}$, $MC_{GAS}$, $MC_{PAR}$) and the running mean over the long period ($ML_{COV}$, $ML_{GAS}$, $ML_{PAR}$).

3. Method for detecting a fault in an electrical enclosure according to the preceding claim, **characterized in that** the short period (SP) is between 15 and 60 minutes and the long period (LP) is between 5 and 12 hours.

4. Method for detecting a fault in an electrical enclosure (10) according to one of the preceding claims, **characterized in that** calculating (270, 370, 470) a change of concentration, respectively, of volatile organic compounds ($EV_{VOC}$), of gases ($EV_{gas}$) and of microparticles ($EV_{par}$) comprises at least one calculation of the difference between two consecutive measurements of the concentration, respectively, of volatile organic compounds ($MES_{COV}$), of gases ($MES_{gas}$) and of microparticles ($MES_{par}$).

5. Device (20) for detecting an electrical fault comprising:

- at least one sensor (21) for supplying a signal characteristic of the concentration of volatile organic compounds;
- at least one sensor (22) for supplying a signal characteristic of the concentration of microparticles;
- at least one sensor (23) for supplying a signal characteristic of the concentration of gases;
- at least one climate parameter sensor (24) for supplying a value of at least one climate parameter (T);
- a measurement circuit (25) for measuring the signals supplied by the sensors (21, 22, 23, 24);
- an alarm circuit (26) for generating an alarm (AL); and
- a processing unit (27),

**characterized in that** the processing unit (27) comprises circuits suitable for executing the method for detecting a fault according to Claims 1 to 4 and for activating the alarm circuit (26) when:

- the drift of concentration threshold ($SD_{par}$) of microparticles or the change of concentration threshold ($EV_{par}$) of microparticles is exceeded; and when
- at least one drift of concentration or change of concentration threshold of volatile organic compounds ($SD_{VOC}$, $EV_{VOC}$) or of gases ($SD_{gas}$, $EV_{gas}$) is exceeded.

6. Device (20) for detecting an electrical fault according to the preceding claim, **characterized in that** the gas sensor (23) supplies a signal characterizing a concentration of ammonia.

7. Device (20) for detecting an electrical fault according to either of Claims 5 and 6, **characterized in that** the gas sensor (23) supplies a signal characterizing a concentration of ozone.

8. Device (20) for detecting an electrical fault according to one of Claims 5 to 7, **characterized in that** the volatile organic compounds sensor (21) supplies a signal characterizing a concentration of hydrocarbons.

9. Device (20) for detecting an electrical fault according to one of Claims 5 to 8, **characterized in that** the climate parameter sensor (24) supplies a signal characterizing a temperature (T) in the electrical enclosure (10).

10. Device (20) for detecting an electrical fault according to Claim 9, **characterized in that** the climate parameter sensor (24) also supplies a signal characterizing humidity (H) in the electrical enclosure (10).

11. Electrical enclosure (10) comprising at least one cable or one electrical equipment item (11, 12), **characterized in that** it comprises a device (10) for detecting an electrical fault according to one of Claims 5 to 10.

**FIG.1**

300

310 — MES$_{gaz}$

320 — Correction MES$_{gaz}$

330 — ML$_{gaz}$

340 — MC$_{gaz}$

350 — DR$_{gaz}$ = MC$_{gaz}$ / ML$_{gaz}$

370 — Calcul EV$_{gaz}$

360

étape 100

Non — DR$_{gaz}$ > SD$_{gaz}$ — Oui

380

Non — EV$_{gaz}$ > SE$_{gaz}$ — Oui

ALD$_{gaz}$

ALE$_{gaz}$

étape 500

étape 500

**FIG.2**

EP 3 512 056 B1

400

410 — MES_par

420 — Correction MES_par

430 — ML_par

440 — MC_par

450 — DR_par = MC_par / ML_par

460 — 

étape 100

Non — DR_par > SD_par — Oui

470 — Calcul EV_par

480 —

Non — EV_par > SE_par — Oui

ALD_par

ALE_par

étape 500

étape 500

**FIG.3**

15

270

271 — EVcov (t+1)= MEScov (t+1)-MEScov (t)

272 —
Non ← Evcov (t+1) > 0 → Oui

273 — EVcov (t+2)= MEScov (t+2)-MEScov (t+1)

274 —
Non ← Evcov (t+2) > 0 → Oui

275 — EVcov (t+3)= MEScov (t+3)-MEScov (t+2)

276 —
Non ← Evcov (t+3) > 0 → Oui

277 — EVcov = MEScov (t+3)-MEScov (t)

280 —
Non ← EVcov > SEcov → Oui
ALEcov

Etape 100                    Etape 500

**FIG.4**

**FIG.5**

EVcov                                        concentration COV

▲ MES cov       ✖ Evcov (t+1) = MEScov(t+1) - MEScov (t)

● EVcov > 0        ▬ ALEcov

temps

**FIG.6**

**FIG.7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6317053 B1
- EP 1768074 A1
- WO 2010043272 A
- EP 0660282 B1
- EP 0141987 B1